# EUROPÄISCHE PATENTSCHRIFT

(11) **EP 2 681 531 B1**
(45) Veröffentlichungstag und Bekanntmachung des Hinweises auf die Patenterteilung: **17.03.2021**
(21) Anmeldenummer: 12707727.9
(22) Anmeldetag: 27.02.2012
(51) Int. Cl.: G01N 21/05, A61L 2/08

(54) **DURCHSTRÖMBARE MESSZELLE ZUR AUFNAHME VON MESSMITTELN**
PERMEABLE MEASURING CELL FOR RECEIVING MEASURING MEANS
CELLULE DE MESURE À CIRCULATION POUR LA RÉCEPTION D'ÉLÉMENTS DE MESURE

(30) Priorität: 04.03.2011 DE 102011013001
(43) Veröffentlichungstag der Anmeldung: 08.01.2014
(60) Teilanmeldung: 21154581.9
(73) Patentinhaber: optek-Danulat GmbH, 45356 Essen (DE)
(72) Erfinder: PLATTE, Daniel, 42555 Velbert (DE); SCHROEREN, Peter, 47906 Kempen (DE); DANULAT, Jürgen, 40822 Mettmann (DE); REESE, Andreas, 45141 Essen (DE)
(74) Vertreter: Schneider, Sascha
(86) Internationale Anmeldenummer: PCT/EP2012/053254
(87) Internationale Veröffentlichungsnummer: WO 2012/119876

(56) Entgegenhaltungen:
- EP-A1- 0 089 157
- GB-A- 2 282 880
- GB-A- 2 446 934

## Beschreibung

Die vorliegende Erfindung betrifft eine durchströmbare Messzelle gemäß Patentanspruch 1 sowie ein System gemäß Patentanspruch 12.

Insbesondere in der Biotechnologie und der Lebensmitteltechnologie sind durchströmbare Messzellen zur Steuerung und Validierung sowie Einhaltung von bestimmten Vorgaben nicht mehr wegzudenken. Anwendungsbeispiele sind die Chromatographie oder Ultrafiltration.

Besonders wichtig ist die Genauigkeit der Messzellen bei der Messung und das schnelle Ansprechen, weshalb solche Messzellen bisher aus qualitativ sehr hochwertigen Materialen hergestellt werden, beispielsweise Edelstahl. Ein wichtiger Aspekt besteht auch in der Reinigungsmöglichkeit, zumal die Messzelle häufig inline eingesetzt werden.

Da häufig, insbesondere in der Biotechnologie, sehr teuere Fluide untersucht werden, spielt auch das Volumen der Messzelle sowie entsprechende Toträume eine große Rolle. So besteht die Bestrebung, das Volumen des Messraums der Messzellen möglichst zu reduzieren, um beispielsweise eine Verschleppung bei einem Phasenwechsel und entsprechenden Materialverbrauch der teueren Medien möglichst gering zu halten.

Entscheidend ist aber auch die Leerlaufeigenschaft der Messzelle, damit nach Ende des Messvorgangs keine Reste des Fluids mehr im Messraum verbleiben. Nicht nur die Reinigung spielt eine entscheidende Rolle, sondern auch die Möglichkeit einer Sterilisierung.

Die GB2282880A zeigt eine Vorrichtung zum Messen von Eigenschaften einer Flüssigkeit. Die GB2446934A offenbart eine Messzelle, die mit Gammastrahlen sterilisierbar ist. Die EP0089157A1 zeigt eine optische Detektorzelle.

Aufgabe der vorliegenden Erfindung ist es daher, eine nach den vorgenannten Vorgaben optimierte durchströmbare Messzelle anzugeben.

Diese Aufgabe wird mit den Merkmalen der Patentansprüche 1 und 12 gelöst. Vorteilhafte Weiterbildungen der Erfindung sind in den Unteransprüchen angegeben. In den Rahmen der Erfindung fallen auch sämtliche Kombinationen aus zumindest zwei von in der Beschreibung, den Ansprüchen und/oder den Figuren angegebenen Merkmalen. Bei angegebenen Wertbereichen sollen auch innerhalb der genannten Grenzen liegende Werte als Grenzwerte offenbart gelten und in beliebiger Kombination beanspruchbar sein.

Der Erfindung liegt der Gedanke zugrunde, eine Messzelle anzugeben, in der bei geringst möglichem Volumen des Messraums sowohl eine Messung mit elektromagnetischer Strahlung als auch eine Leitfähigkeitsmessung und/oder eine pH-Messung und/oder eine Temperaturmessung durchführbar sind. Somit werden mindestens zwei Messungen in einem Messraum durch die erfindungsgemäße Ausgestaltung der durchströmbaren Messzelle ermöglicht, wovon eine eine Strahlungsmessung mit elektromagnetischer Strahlung ist. Insbesondere für die Strahlungsmessung ist zur Ermittlung der Wechselwirkung des Fluids mit der elektromagnetischen Strahlung ein gewisser Strahlengang erforderlich, sodass eine Reduzierung des Volumens kaum möglich ist. Die Erfindung besteht daher darin, mindestens eine weitere Messung im selben Messraum vorzusehen, um das bisher erforderliche Volumen für beide Messungen insgesamt sowie die Anzahl der zu verbauenden Messzellen zu reduzieren.

Gemäß einer vorteilhaften Ausführungsform der Erfindung ist vorgesehen, dass die Messzelle zumindest überwiegend, insbesondere zu mindestens 90%, vorzugsweise zu mindestens 95%, aus chemischen Elementen mit einer Ordnungszahl < 17 besteht. Somit ist die Messzelle so weit gammadurchlässig, dass eine vollständige und homogene Beaufschlagung des Messraums mit Gammastrahlen zur Desinfektion ermöglicht wird. Auf diese Weise wird die Herstellung und der Versand beziehungsweise Transport der erfindungsgemäßen Messzellen stark vereinfacht, da die Messzellen im verpackten Zustand mit Gammastrahlen beaufschlagt und entsprechend desinfiziert werden können. Somit kann eine Kontamination bei der Verpackung der Messzellen ausgeschlossen werden und die Verpackung entsprechend kostengünstiger durchgeführt werden.

Indem die Messzelle einen Temperaturmessbereich zur Anordnung, insbesondere zum Anschluss, eines Temperatursensors aufweist, lässt sich zusätzlich und auf einfache Art und Weise eine Temperaturmessung in die Messzelle integrieren.

Die Erfindung geht gemäß einer vorteilhaften Ausführungsform der Erfindung den zum Stand der Technik entgegengesetzten Weg, indem die Messzelle als Einwegmesszelle, insbesondere überwiegend, vorzugsweise im Wesentlichen vollständig, aus Kunststoff ausgebildet ist. Auf diese Weise ist es möglich, die für die Messung erforderlichen, hochwertigen und teueren Messmittel, für die besonders hohe Qualitätsanforderungen gelten, nach jedem Zyklus oder jeweils nach einer bestimmten Zeitdauer oder sogar bei jedem Wechsel eines Fluids, auszutauschen, während die teuren Messmittel weiterverwendet werden können.

Besonders vorteilhaft ist es bei der vorliegenden Erfindung, dass gemäß einer Ausführungsform der Erfindung der, insbesondere überwiegend röhrenförmige, Messraum ein Volumen von weniger als 50ml, insbesondere weniger als 30ml, aufweist. Somit werden auf kleinstem Raum eine Vielzahl von Messungen an dem die Messzelle durchströmenden Fluid ermöglichst und der Materialverbrauch beziehungsweise die Verschleppung beim Phasenwechsel minimiert.

Indem die Eintrittsöffnung und die Austrittsöffnung parallel versetzt zueinander verlaufen, lässt sich die Messzelle optimal in bestehende Systeme einbauen. Hierdurch wird außerdem die Montage der Messzelle erleichtert.

Ein besonders gutes Strömungsprofil mit optimalem Leerlaufverhalten ist realisierbar, indem die Messzelle derart gestaltet ist, dass das Fluid von der Eintrittsöffnung bis zur Austrittsöffnung mindestens eine, insbesondere zwei, vorzugsweise genau zwei, Krümmungen durchläuft. Die Krümmungen haben insbesondere einen Krümmungswinkel von mindestens 45°, vorzugsweise etwa 90°. Auf diese Weise werden an der Messzelle mehrere Freiflächen zur Anbringung von Messmitteln geschaffen.

Gemäß einer weiteren, vorteilhaften Ausführungsform der Erfindung ist vorgesehen, dass ein Strahlengang des Strahlungsmessbereichs quer zum Messraum und quer zur Eintrittsöffnung und/oder Austrittsöffnung verläuft. Auf diese Weise wird die Strahlungsmessung mit geringst möglichem Platzbedarf in die Messzelle oder an der Messzelle verwirklicht.

Für die Leitfähigkeitsmessaufnahme und/oder die pH-Messaufnahme gilt gemäß einer weiteren vorteilhaften Ausführungsform der Erfindung, dass diese längs zur Messform und quer zur Eintrittsöffnung und/oder Austrittsöffnung angeordnet ist/sind. Hierdurch lässt sich unter optimaler Platzausnutzung eine Vollintegration der genannten Messmittel bei geringst möglichen Volumen erreichen.

Das erfindungsgemäße System wird dadurch optimiert, indem gemäß einer vorteilhaften Ausführungsform die Messzelle als Einwegmesszelle für einen Messzyklus verwendbar ist beziehungsweise verwendet wird, während die Strahlungsmessmittel und/oder die Leitfähigkeitsmessmittel und/oder die pH-Wert-Messmittel für mehrere Messzyklen verwendbar sind beziehungsweise verwendet werden.

Weitere Vorteile, Merkmale und Einzelheiten der Erfindung ergeben sich aus der nachfolgenden Beschreibung bevorzugter Ausführungsbeispiele sowie anhand der Zeichnungen. Diese zeigen in:
- Figur 1: eine perspektivische Ansicht einer erfindungsgemäßen Messzelle mit Schnittebene A und Schnittebene B,

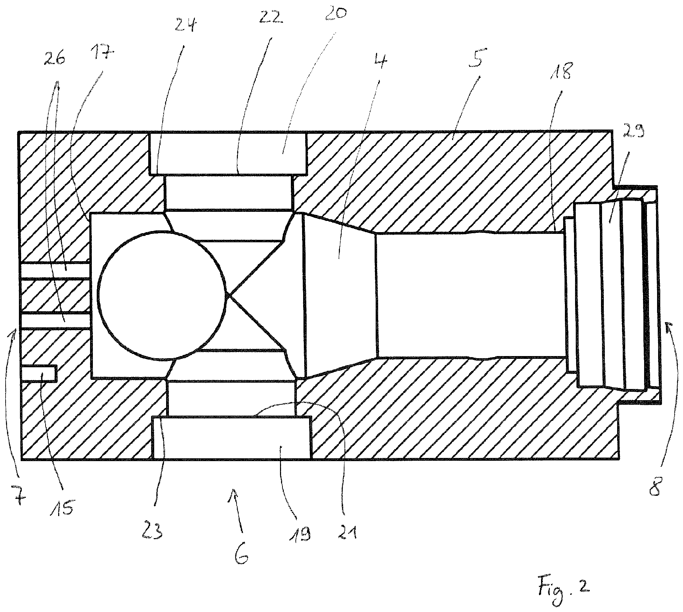

- Figur 2: eine geschnittene Ansicht der Messzelle gemäß Schnittebene A aus Figur 1 und
- Figur 3: eine geschnittene Ansicht der Messzelle der Schnittebene B aus Figur 1.

In den Figuren sind gleiche oder gleich wirkende Elemente oder Elemente mit der gleichen Funktion mit dem gleichen Bezugszeichen gekennzeichnet.

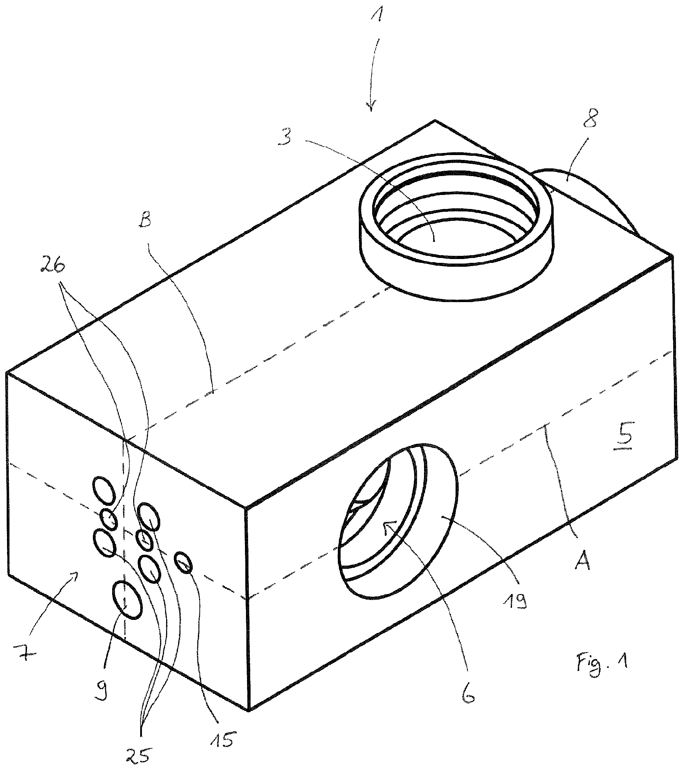

Figur 1 zeigt eine im Wesentlichen quaderförmige, durchströmbare Messzelle 1 mit diversen, unten beschriebenen Mitteln zur Aufnahme von Messmitteln zur Messung von chemischen und/oder physikalischen Eigenschaften eines die Messzelle 1 durchströmenden Fluids.

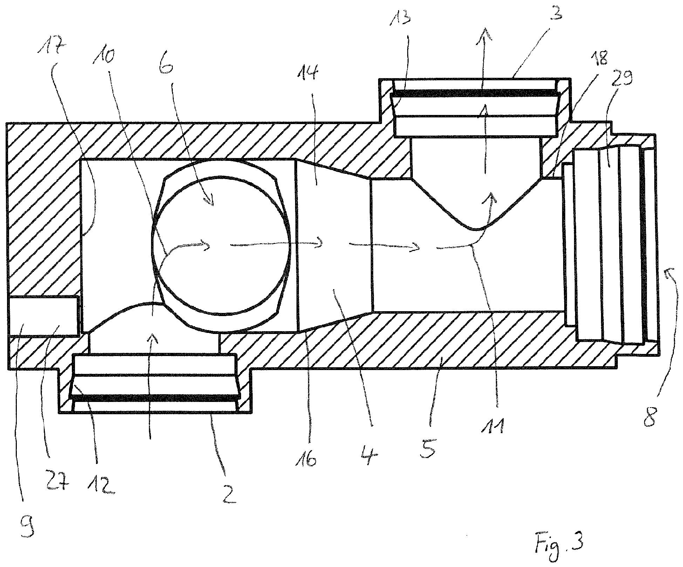

In Figur 3 ist anhand von schematischen Pfeilen (Strömungsverlauf) zu erkennen, dass das Fluid durch eine Eintrittsöffnung 2 zum Einlauf des Fluids in einen Messraum 4 gelangt. Der Messraum 4 erstreckt sich in einem Winkel von 90° zu der Eintrittsöffnung 2 nach rechts, so dass das Fluid einer Krümmung 10 und somit einer schematisch durch einen Pfeil dargestellten Kurve folgt. Nachdem das Fluid durch den Messraum 4 hindurch geströmt ist, läuft das Fluid durch eine Austrittsöffnung 3 aus der Messzelle 1 heraus. Kurz vor der Austrittsöffnung 3 ist eine in die entgegengesetzte Richtung der Krümmung 10 verlaufende Krümmung 11 vorgesehen, sodass das Fluid wiederum einer durch einen Pfeil dargestellte Kurve um 90° folgt.

Sowohl an der Eintrittsöffnung 2 als auch an der Austrittsöffnung 3 sind Anschlussmittel 12, 13 vorgesehen, über welche die Messzelle 1 an entsprechende Anschlüsse in den Prozesslauf als inline-Messzelle anschließbar ist. Im mittleren Bereich des Messraums 4 ist eine, insbesondere konusförmige, Reduzierung 14 des Messraums 4 vorgesehen, um eine möglichst blasenfreie oder laminare Strömung des Fluids zu gewährleisten. An den Anschlussmitteln 12, 13 sind Dichtungsmittel vorgesehen. Für die Anschlussmittel 12, 13 sind mit Vorteil, insbesondere als Einwegadapter ausgebildete, Adapter zum, insbesondere steckbaren, Anschluss verschiedener Leitungsanschlüsse vorgesehen. Die Adapter sind insbesondere aus Kunststoff gebildet und werden gleichzeitig mit der Messzelle verpackt und als Messzellenset zur Verfügung gestellt. Ein solches Messzellenset bietet den Vorteil, dass der inline-Einbau problemlos, schnell und sicher an verschiedene Leitungssysteme erfolgen kann und somit auch die Lagerhaltungskosten reduziert werden.

Die Messzelle 1 besteht im Wesentlichen aus einem einteiligen Messzellenkörper 5 aus Kunststoff, insbesondere Polyphenylensulfon. Erfindungsgemäße Eigenschaften des Kunststoffs sind: präzise Bearbeitbarkeit, hohe Steifheit, Gammadurchlässigkeit und hohe Verbrennbarkeit, d.h. zu mindestens 95% der Masse in der Zeit üblichen Prozessen der Hausmüllverbrennung die Gasphase überführbar.

Durch Vorsehen einer Kodierung 15 ist die Messzelle 1, insbesondere automatisch, mit den Messmitteln unter Vermeidung eines verdrehten oder falschen Anschlusses koppelbar. Hierzu sind an der anzuschließenden Leitung oder einer Aufnahme für die Messzelle 1 an der Leitung korrespondierende Koppelmittel vorgesehen.

Die Kodierung 15 oder eine zusätzliche Kodierung umfasst in einer vorteilhaften Ausführungsform der Erfindung eine Parameterkennung für einen oder mehrere Parameter der Messzelle 1. Die Parameterkennung kann aus einer geometrischen Ausbildung der Kodierung 15 oder der zusätzlichen Kodierung bestehen, die von den Koppelmittel oder separaten Erfassungsmitteln erfasst werden. Besonders vorteilhaft ist eine mechanische oder elektronische Parameterkennung. Als elektronische Parameterkennung kommt insbesondere ein Transponder für die Identifizierung mit Hilfe elektromagnetischer Wellen in Frage.

Die Parameter sind insbesondere die Zellkonstante für die Leitfähigkeitsmessung und/oder die optische Pfadlänge der jeweiligen Messzelle 1.

Der Messraum 4 weist einen, insbesondere röhrenförmigen (vorzugsweise mit kreisförmigen Querschnitt), Messkanal 16 auf, der sich quasi über die gesamte Länge der Messzelle 1 erstreckt. An einem ersten Ende 17 des Messkanals 16 ist die Eintrittsöffnung 2 am Messkanal 16 angewinkelt angeordnet, während an einem zweiten Ende 18 des Messkanals 16 die Austrittsöffnung 3 angewinkelt angeordnet ist, und zwar in entgegengesetzte Richtung zur Eintrittsöffnung 2.

Die Axialrichtung der Eintrittsöffnung 2 und die Axialrichtung der Austrittöffnung 3 sind parallel zueinander und verlaufen quer beziehungsweise unter einem Winkel von 90° zur Axialrichtung des Messkanals 16.

Quer beziehungsweise unter einem Winkel von 90° zum Messkanal sowie insbesondere auch quer beziehungsweise unter einem Winkel von 90° zu der Axialrichtung der Eintrittsöffnung 2 beziehungsweise der Austrittsöffnung 3 liegt ein Strahlenmessbereich 6 zur Messung der Wechselwirkung des Fluids in der Messzelle 1 mit einer elektromagnetischen Strahlung. Elektromagnetische Strahlung tritt von einer nicht dargstellten Strahlungsquelle durch eine Strahlungseintrittsöffnung 19 in den Messraum 4 ein und auf der gegenüberliegenden Seite durch eine Strahlungsaustrittsöffnung 20 wieder aus dem Messraum 4 heraus, wo sie auf eine Strahlungsmesseinrichtung trifft. Der Strahlengang verläuft quer beziehungsweise in einem Winkel von 90° zu dem Messkanal 16 und der Eintrittsöffnung 2 beziehungsweise der Austrittsöffnung 3. Die Axialrichtung der zueinander fluchtenden Strahlungseintrittsöffnung 19 und Strahlungseintrittsöffnung 20 deckt sich mit dem Strahlengang.

In der Strahleneintrittsöffnung 19 und in der Strahlenaustrittöffnung 20 sind jeweils Fensteraufnahmen 21, 22 zur Aufnahme von für die elektromagnetische Strahlung der Strahlungsquelle transparenten Fenstern vorgesehen. Die Fenster dichten den Messraum 4 gegenüber der Umgebung ab.

Die Messzelle 4 ist erfindungsgemäß so ausgebildet, dass im Strahlengang zumindest zwischen zwischen der Strahleneintrittsöffnung 19 und der Strahlenaustrittsöffnung 20, insbesondere zwischen den Fenstern, keine die Messung störenden weiteren Bauteile angeordnet sind.

Die optische Pfadlänge, also die Strecke, die die elektromagnetische Strahlung beim Durchtritt durch das Fluid zurücklegt, ergibt sich durch die Anlage der Fenster an Anschlägen 23, 24 der Fensteraufnahme 21, 22.

An dem ersten Ende 17 ist eine Leitfähigkeitmessaufnahme 7 zur Aufnahme von Leitfähigkeitsmessmitteln zur Messung der Leitfähigkeit des Fluids in der Messzelle 1 vorgesehen. Die Leitfähigkeitsmessaufnahme 7 besteht im vorliegenden Fall aus vier Aufnahmeöffnungen 25 für Stromelektroden und zwei zwischen den Aufnahmeöffnungen 25 angeordneten Aufnahmeöffnungen 26 für Spannungselektroden. In den Aufnahmeöffnungen 25, 26 sind die Strom- beziehungsweise Spannungselektroden dichtend aufnehmbar, sodass diese möglichst plan mit dem ersten Ende 17 abschließen oder leicht in den Messraum 4 hineinragen. Die Funktion eines Leitfähigkeitssensors ist in der DE 19946315C2 beschrieben. In einer vorteilhaften Ausführungsform der Erfindung wird der Leitfähigkeitssensor gemäß DE19946315C2 in einer Aufnahmeöffnung zur Aufnahme des Gehäuses 1 des Leitfähigkeitssensors gemäß DE19946315C2 geeignet und in Kombination mit diesem als Erfindung mitoffenbart.

Neben (vorzugsweise an der gleichen Seite der Messzelle 1) der Leitfähigkeitsmessaufnahme 7 ist eine mechanische Kodierung 15 vorgesehen, die mit einem entsprechenden Stift eine Aufnahmeeinrichtung beziehungsweise Koppeleinrichtung zur Kopplung der Messzelle 1 an der Prozessleitung vorgesehen, wobei an dem Messzellenkörper 5 mehrere, insbesondere asymmetrisch an dem Messzellenkörper 5 verteilte, Kodierungen 15 vorgesehen sein können.

Ebenfalls neben (vorzugsweise an der gleichen Seite der Messzelle 1) der Leitfähigkeitsmessaufnahme 7 ist ein Temperaturmessbereich 9 in Form eines fast bis zum Messraum 4 reichenden Sacklochs 27 vorgesehen. Das Sackloch 27 endet in unmittelbarer Nähe des ersten Endes 17 und im Bereich der Eintrittsöffnung 2. Zwischen dem Sackloch 27 und dem erstem Ende 17 ist eine dünne Trennwand vorgesehen, durch die eine Messspitze eines Temperaturfühlers hindurch gestochen werden kann, sodass eine verlässliche Messung und gleichzeitig eine gute Abdichtung gegenüber der Umgebung ermöglicht wird.

Am gegenüberliegenden zweiten Ende 18 ist eine pH-Messaufnahme 8 zur Aufnahme von pH-Wert-Messmitteln zur Messung des pH-Werts des Fluids in der Messzelle 1 vorgesehen. Die pH-Messaufnahme 8 umfasst eine Aufnahmeöffnung 29, deren Axialrichtung mit der Axialrichtung des Messkanals 16 fluchtend ausgerichtet ist. Ein in die Aufnahmeöffnung 29 steckbare pH-Elektrode ist somit dichtend gegenüber der Umgebung der Messzelle 1 in den Messraum 4 steckbar und misst den pH-Wert des vorbeiströmenden Fluids.

Eine Spitze der pH-Elektrode ist bei dem erfindungsgemäßen System mit Vorteil derart in der pH-Messaufnahme 8 anbringbar, dass diese mindestens bis unter der Austrittsöffnung 3, insbesondere mindestens bis zur Mitte der Austrittsöffnung 3 in dem Messkanal 16 reicht. Die pH-Elektrode ist an der Aufnahmeöffnung 29 dichtend fixierbar.

Die Messzelle 1 ist erfindungsgemäß so horizontal ausrichtbar, wie es in den Figuren dargestellt ist, so dass die Eintrittsöffnung 2 und/oder die Austrittsöffnung 3 mit der Normalen fluchten. Hierdurch wird ein optimales Leerlaufverhalten erreicht.

Der Bauraum der Messzelle 1 wird weiter minimiert, wenn dabei ein Strahlengang für die Messung der elektromagnetischen Strahlung (Strahlungsmessbereich 6) horizontal verläuft.

Noch weiter optimierbar ist dies, indem die pH-Messaufnahme im Wesentlichen horizontal, insbesondere maximal mit einem Winkel von 20° zur Horizontalen, verläuft.

### Bezugszeichenliste

- 1: Messzelle
- 2: Eintrittsöffnung
- 3: Austrittsöffnung
- 4: Messraum
- 5: Messzellenkörper
- 6: Strahlungsmessbereich
- 7: Leitfähigkeitsmessaufnahme
- 8: pH-Messaufnahme
- 9: Temperaturmessbereich
- 10: Krümmung
- 11: Krümmung
- 12: Anschlussmittel
- 13: Anschlussmittel
- 14: Reduzierung
- 15: Kodierung
- 16: Messkanal
- 17: Erstes Ende
- 18: Zweites Ende
- 19: Strahlungseintrittsöffnung
- 20: Strahlungsaustrittsöffnung
- 21: Fensteraufnahme
- 22: Fensteraufnahme
- 23: Anschläge
- 24: Anschläge
- 25: Aufnahmeöffnungen
- 26: Aufnahmeöffnungen
- 27: Sackloch
- 29: Aufnahmeöffnung

## Patentansprüche

1. Durchströmbare Messzelle zur Aufnahme von Messmitteln zur Messung von chemischen und/oder physikalischen Eigenschaften eines die Messzelle (1) durchströmenden Fluids mit:
- einem zwischen einer Eintrittsöffnung (2) zum Einlauf des Fluids und einer Austrittsöffnung (3) zum Auslauf des Fluids angeordneten Messraum (4) mit einem sich von einem ersten Ende (17) bis zu einem gegenüberliegenden zweiten Ende (18) ersteckenden Messkanal (16),
- einem Strahlungsmessbereich (6) zur Messung der Wechselwirkung des Fluids im Messraum (4) mit einer elektromagnetischen Strahlung von außerhalb des Messraums (4) und
- einer Leitfähigkeitsmessaufnahme (7) zur Aufnahme von Leitfähigkeitsmessmitteln zur Messung der Leitfähigkeit des Fluids im Messraum (4), wobei die Eintrittsöffnung (2) am ersten Ende (17) am Messkanal (16) angewinkelt angeordnet ist, wobei die Austrittsöffnung (3) am zweiten Ende (18) am Messkanal (16) angewinkelt angeordnet ist, wobei die Messzelle einen Temperaturmessbereich zur Anordnung eines Temperatursensors aufweist, **dadurch gekennzeichnet, dass** die Leitfähigkeitsmessaufnahme (7) am ersten Ende (17) angeordnet ist.

2. Messzelle nach Anspruch 1, bei der die Messzelle (1) zumindest überwiegend, insbesondere zu mindestens 90%, vorzugsweise zu mindestens 95%, aus chemischen Elementen mit einer Ordnungszahl <17 besteht.

3. Messzelle nach einem der vorhergehenden Ansprüche, bei dem die Messzelle (1) als Einwegmesszelle, insbesondere überwiegend, vorzugsweise im Wesentlichen vollständig, aus Kunststoff ausgebildet ist.

4. Messzelle nach einem der vorhergehenden Ansprüche, bei der die Messzelle einen Temperaturmessbereich (9) zum Anschluss eines Temperatursensors aufweist.

5. Messzelle nach einem der vorhergehenden Ansprüche, bei der der, insbesondere überwiegend röhrenförmige, Messraum (4) ein Volumen von weniger als 50ml, insbesondere weniger als 30ml, aufweist.

6. Messzelle nach einem der vorhergehenden Ansprüche, bei der die Eintrittsöffnung (2) und die Austrittsöffnung (3) parallel versetzt zueinander verlaufen.

7. Messzelle nach einem der vorhergehenden Ansprüche, bei der die Messzelle (1) derart gestaltet ist, dass das Fluid von der Eintrittsöffnung (2) bis zur Austrittsöffnung (3) mindestens eine, insbesondere zwei, vorzugsweise genau zwei, Krümmungen (10, 11), insbesondere mit einem Krümmungswinkel von mindestens 45 °, vorzugsweise etwa 90°, durchläuft.

8. Messzelle nach einem der vorhergehenden Ansprüche, bei der ein Strahlengang des Strahlungsmessbereichs (6) quer zum Messraum (4) und quer zur Eintrittsöffnung (2) und /oder Austrittsöffnung (3) verläuft.

9. Messzelle nach einem der vorhergehenden Ansprüche, bei der die Leitfähigkeitsmessaufnahme (7) und/oder die pH-Messaufnahme (8) längs zum Messraum (4) und quer zur Eintrittsöffnung (2) und/ oder der Austrittsöffnung (3) angeordnet ist/sind.

10. Messzelle nach einem der vorhergehenden Ansprüche, bei der die elektromagnetische Strahlung durch eine Strahlungseintrittsöffnung (19) in den Messraum (4) ein und auf der gegenüberliegenden Seite durch eine Strahlungsaustrittsöffnung (20) wieder aus dem Messraum (4) austritt.

11. Messzelle nach Anspruch 10, bei der in der Strahleneintrittsöffnung (19) und in der Strahlenaustrittöffnung (20) jeweils Fensteraufnahmen (21, 22) zur Aufnahme von für die elektromagnetische Strahlung der Strahlungsquelle transparenten Fenstern vorgesehen sind.

12. System aus einer Messzelle nach einem der vorhergehenden Ansprüche und
- einem an der Messzelle anbringbaren Strahlungsmittel zur Erzeugung der elektromagnetischen Strahlung zur Messung der Wechselwirkung des Fluids in der Messzelle (1) mit einer elektromagnetischen Strahlung von außerhalb der Messzelle (1) und
- dem an der Leitfähigkeitsmessaufnahme (7) anbringbaren Leitfähigkeitsmessmittel

13. System nach Anspruch 12, das eine an einer dem Strahlungsmittel gegenüberliegenden Seite der Messzelle anbringbare Strahlungsmesseinrichtung aufweist.

## Claims

1. A flow-through measurement cell for accommodating measuring devices for measuring chemical and/or physical properties of a fluid flowing through the measurement cell (1) with:
- a measurement chamber (4) arranged between an inlet opening (2) for the inflow of the fluid and an outlet opening (3) for the outflow of the fluid with a measurement channel (16) extending from a first end (17) up to an opposite second end (18),
- a radiation measurement area (6) for measuring the interaction of the fluid in the measurement chamber (4) with electromagnetic radiation from outside the measurement chamber (4) and
- a conductivity measurement socket (7) for accommodating conductivity measuring devices for measuring the conductivity of the fluid in the measurement chamber (4), wherein
the inlet opening (2) is arranged at the first end (17) at an angle to the measurement channel (16), wherein the outlet opening (3) is arranged at the second end (18) at an angle to the measurement channel (16), wherein the measurement cell has a temperature measurement area for the arrangement of a temperature sensor, **characterized in that** the conductivity measurement socket (7) is arranged at the first end (17).

2. The measurement cell according to claim 1, wherein the measurement cell (1) is predominantly, in particular at least 90% thereof, preferably at least 95% thereof, made of chemical elements with an atomic number <17.

3. The measurement cell according to one of the preceding claims, wherein the measurement cell (1) is configured as a disposable measurement cell, in particular predominantly, preferably essentially completely, made of plastic.

4. The measurement cell according to one of the preceding claims, wherein the measurement cell has a temperature measurement area (9) for connecting a temperature sensor.

5. The measurement cell according to one of the preceding claims, wherein the, in particular predominantly tube-shaped, measurement chamber (4) has a volume of less than 50ml, in particular less than 30ml.

6. The measurement cell according to one of the preceding claims, wherein the inlet opening (2) and the outlet opening (3), which are offset from one another, are parallel.

7. The measurement cell according to one of the preceding claims, wherein the measurement cell (1) is formed in such a manner that, when flowing from the inlet opening (2) to the outlet opening (3), the fluid flows through at least one, in particular exactly two, bends (10, 11), in particular with an angle of curvature of at least 45°, preferably approximately 90°.

8. The measurement cell according to one of the preceding claims, wherein a radiation path of the radiation measurement area (6) runs transversely to the measurement chamber (4) and transversely to the inlet opening (2) and/or outlet opening (3).

9. The measurement cell according to one of the preceding claims, wherein the conductivity measurement socket (7) and/or the pH measurement socket (8) is/are arranged longitudinally in relation to the measurement chamber (4) and transversely to the inlet opening (2) and/or the outlet opening (3).

10. The measurement cell according to one of the preceding claims, wherein the electromagnetic radiation enters the measurement chamber (4) through a radiation inlet opening (19) and exits the measurement chamber (4) on the opposite side through a radiation outlet opening (20).

11. The measurement cell according to claim 10, wherein window embrasures (21, 22) for the accommodation of windows that are transparent for the electromagnetic radiation of the radiation source are respectively provided in the radiation inlet opening (19) and in the radiation outlet opening (20).

12. A system consisting of a measurement cell according to one of the preceding claims and
- a radiation device that is attachable to the measurement cell for generating the electromagnetic radiation for the measurement of the interaction of the fluid in the measurement cell (1) with electromagnetic radiation from outside the measurement cell (1) and
- the conductivity measuring device that is attachable to the conductivity measurement socket (7).

13. The system according to claim 12, comprising a radiation measuring apparatus that is attachable on a side of the measurement cell that lies opposite the radiation device.

## Revendications

1. Cellule de mesure, susceptible d'être traversée, destinée à recevoir des instruments de mesure pour mesurer des propriétés chimiques et/ou physiques d'un fluide circulant à travers la cellule de mesure (1), comprenant :
- un espace de mesure (4) placé entre un orifice d'entrée (2) pour l'arrivée du fluide et un orifice de sortie (3) pour l'écoulement du fluide, pourvu d'un canal de mesure (16) s'étendant d'une première extrémité (17) jusqu'à une deuxième extrémité (18) opposée,
- une zone de mesure des rayonnements (6), destinée à la mesure de l'interaction du fluide dans l'espace de mesure (4) avec un rayonnement électromagnétique provenant de l'extérieur de l'espace de mesure (4) et
- un support de mesure de conductivité (7), destiné à recevoir des instruments de mesure de conductivité pour mesurer la conductivité du fluide dans l'espace de mesure (4),
l'orifice d'entrée (2) étant placé de manière coudée sur la première extrémité (17) sur le canal de mesure (16), l'orifice de sortie (3) étant placé de manière coudée sur la deuxième extrémité (18) sur le canal de mesure (16), la cellule de mesure comportant une zone de mesure de la température, destinée à y placer un capteur de mesure, **caractérisée en ce que** le support de mesure de conductivité (7) est placé sur la première extrémité (17).

2. Cellule de mesure selon la revendication 1, sur laquelle la cellule de mesure (1) est majoritairement constituée, notamment à raison d'au moins 90 %, de préférence, à raison d'au moins 95 %, d'éléments chimiques de numéro atomique < 17.

3. Cellule de mesure selon l'une quelconque des revendications précédentes, sur laquelle la cellule de mesure (1) est conçue sous la forme d'une cellule de mesure jetable, notamment majoritairement, de préférence sensiblement en totalité en une matière plastique.

4. Cellule de mesure selon l'une quelconque des revendications précédentes, sur laquelle la cellule de mesure comporte une zone de mesure de la température (9), destinée à y raccorder un capteur de mesure.

5. Cellule de mesure selon l'une quelconque des revendications précédentes, sur laquelle l'espace de mesure (4) majoritairement de forme tubulaire présente un volume inférieur à 50ml, notamment inférieur à 30ml.

6. Cellule de mesure selon l'une quelconque des revendications précédentes, sur laquelle l'orifice d'entrée (2) et l'orifice de sortie (3) s'écoulent à la parallèle en étant mutuellement décalés.

7. Cellule de mesure selon l'une quelconque des revendications précédentes, sur laquelle la cellule de mesure (1) est conçue de telle sorte que le fluide traverse, de l'orifice d'entrée (2) jusqu'à l'orifice de sortie (3) au moins une, notamment deux, de préférence précisément deux courbures (10, 11) présentant notamment un angle de courbure d'au moins 45 °, de préférence d'approximativement 90 °.

8. Cellule de mesure selon l'une quelconque des revendications précédentes, sur laquelle un chemin optique de la zone de mesure des rayonnements (6) s'écoule à la transversale de l'espace de mesure (4) et à la transversale de l'orifice d'entrée (2) et /ou de l'orifice de sortie (3).

9. Cellule de mesure selon l'une quelconque des revendications précédentes, sur laquelle le support de mesure de conductivité (7) et/ou le support de mesure du pH (8) est/sont placé(s) le long de l'espace de mesure (4) et à la transversale de l'orifice d'entrée (2) et/ ou de l'orifice de sortie (3).

10. Cellule de mesure selon l'une quelconque des revendications précédentes, sur laquelle le rayonnement électromagnétique pénètre à travers un orifice d'entrée de rayonnement (19) dans l'espace de mesure (4) et sur le côté opposé, s'échappe de nouveau à travers un orifice de sortie de rayonnement (20) hors de l'espace de mesure (4).

11. Cellule de mesure selon la revendication 10, sur laquelle dans l'orifice d'entrée de rayonnement (19) et dans l'orifice de sortie de rayonnement (20) sont prévus respectivement des supports de fenêtres (21, 22), destinés à recevoir des fenêtres transparentes au rayonnement électromagnétique de la source de rayonnement.

12. Système composé d'une cellule de mesure selon l'une quelconque des revendications précédentes et
- d'un générateur de rayonnement susceptible d'être monté sur la cellule de mesure, destiné à générer le rayonnement électromagnétique pour mesurer l'interaction du fluide dans la cellule de mesure (1) avec un rayonnement électromagnétique provenant de l'extérieur de la cellule de mesure (1) et
- de l'instrument de mesure de conductivité susceptible d'être monté sur le support de mesure de conductivité (7).

13. Système selon la revendication 12, qui comporte un système de mesure de rayonnement susceptible d'être monté sur le côté de la cellule de mesure qui est opposé au générateur de rayonnement.
